# EUROPEAN PATENT APPLICATION

(11) **EP 1 039 312 A2**
(43) Date of publication of application: **27.09.2000**
(21) Application number: 99125028.3
(22) Date of filing: 15.12.1999
(51) Int. Cl.: G01S 7/52

(54) **Ultrasonic imaging system for realizing optimum harmonic image and a method therefor**

(30) Priority: 16.03.1999 KR 9908744
(71) Applicant: MEDISON CO., LTD., Kangwon-do 250-870 (KR)
(72) Inventor: Son, Keon Ho, Kangnam-gu, Seoul, 135-280 (KR); Kim, Jae Gyoung, Kangnam-gu, Seoul, 135-280 (KR); Lee, Min Hwa, Kangnam-gu, Seoul, 135-110 (KR)
(74) Representative: Sama, Daniele, Dr.

(57) **Abstract**

An optimum harmonic imaging (OHI) system for realizing an OHI and a method therefor is provided in which an ultrasonic velocity used for processing an ultrasonic signal, which is transmitted into a human body and is reflected therefrom is varied according to the medium in the human body from which a harmonic image is desired to be obtained. Thus, an OHI can be obtained in which a contrast resolution and a lateral resolution are further improved and an artifact is minimized.

## Description

### BACKGROUND OF THE INVENTION

### 1. Field of the Invention

The present invention relates to an ultrasonic imaging system and a method therefor, and more particularly, to an ultrasonic imaging system for realizing an optimum harmonic image (OHI) in which an ultrasonic velocity used for processing an ultrasonic signal, which is transmitted into a human body and is reflected therefrom is varied according to a medium in the human body from which a harmonic image is obtained.

### 2. Description of the Related Art

Recently, a diagnostic system using an ultrasonic wave is being widely used in a general medical field. The ultrasonic diagnostic system uses an acousto-physical reaction or information such as change in speed of sound, reflection, scattering, absorption, attenuation and the Doppler phenomenon that occur when the ultrasonic wave passes through tissue of a living body.

When an ultrasonic wave is applied to a human body and the returning ultrasonic reception signal is realized into an image, a pulse reflection method, a transmission method, a Doppler method and so on are used. The quality of the ultrasonic image is being greatly improved due to development in performance of a transducer for transmitting and/or receiving an ultrasonic wave and in the technologies for focusing the ultrasonic reception signal and processing a digital signal.

Also, in order to further improve a contrast resolution and a lateral resolution of an ultrasonic image which has been realized in this manner and to minimize its artifact, a harmonic imaging method is being widely used, in which only a harmonic signal is taken to realize an ultrasonic image.

After a transducer has transmitted an ultrasonic wave into a human body, an ultrasonic reception signal coming back from the human body includes a signal (fundamental signal) of the frequency having the substantially same magnitude as that of the frequency of the transmitted signal and a signal (harmonic signal) of the frequency corresponding to an integer multiple magnitude with respect to the frequency of the transmitted signal. A general ultrasonic image (fundamental image) is realized by using a fundamental signal and a harmonic image is realized by using a harmonic signal which is double the frequency of the transmitted signal.

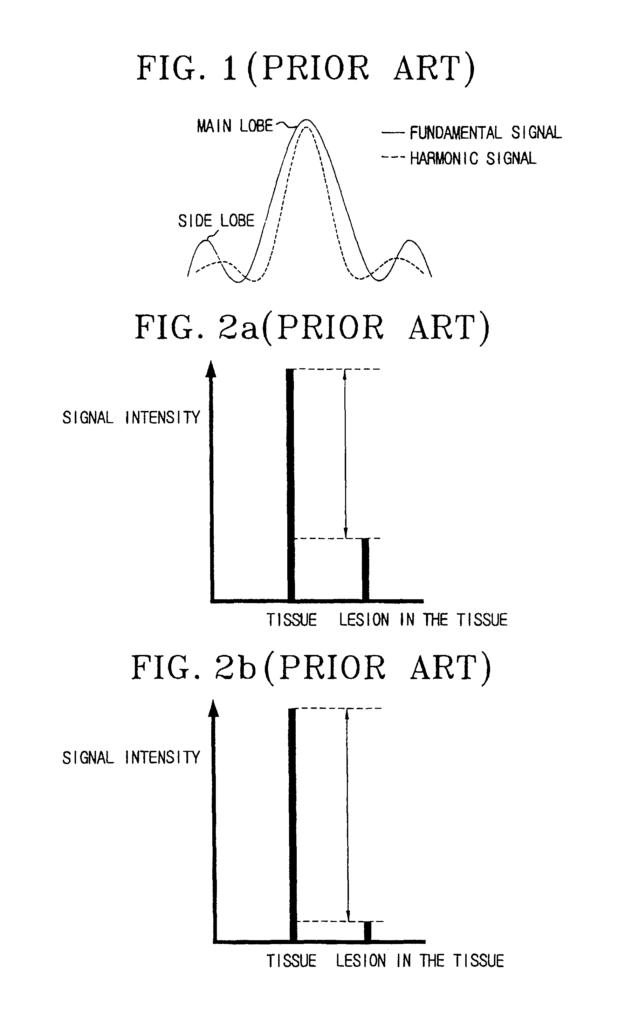

The harmonic signal for the above harmonic image is generated because a non-linear speed difference occurs due to sound pressure when an ultrasonic wave travels in the human body. Comparing the harmonic signal with the fundamental signal, as shown Fig. 1, the harmonic signal has features of a narrower main lobe and a lower side lobe than those of the fundamental signal. Thus, the harmonic image has a reduced artifact and a further improved contrast resolution and lateral resolution, compared with the fundamental image.

In addition, when a lesion in the human body, that is, an abnormality in the tissue in the human body occurs, the difference in the intensities between the harmonic signal from the tissue in the human body and that from the lesion in the tissue therein appears larger than the difference in the intensities between the fundamental signal from the tissue in the human body and that from the lesion in the tissue therein, to thereby improve the contrast resolution, which is shown in FIGs. 2a and 2b.

FIG. 2a illustrates signal intensities of the fundamental signal from the tissue in the human body and that from the lesion in the tissue therein. FIG. 2b illustrates signal intensities of the harmonic signal from the tissue in the human body and that from the lesion in the tissue therein. As shown in FIGs. 2a and 2b, it can be seen that the difference in the intensities between the harmonic signal from the tissue in the human body and that from the lesion in the tissue therein is larger than the difference in the intensities between the fundamental signal from the tissue in the human body and that from the lesion in the tissue therein.

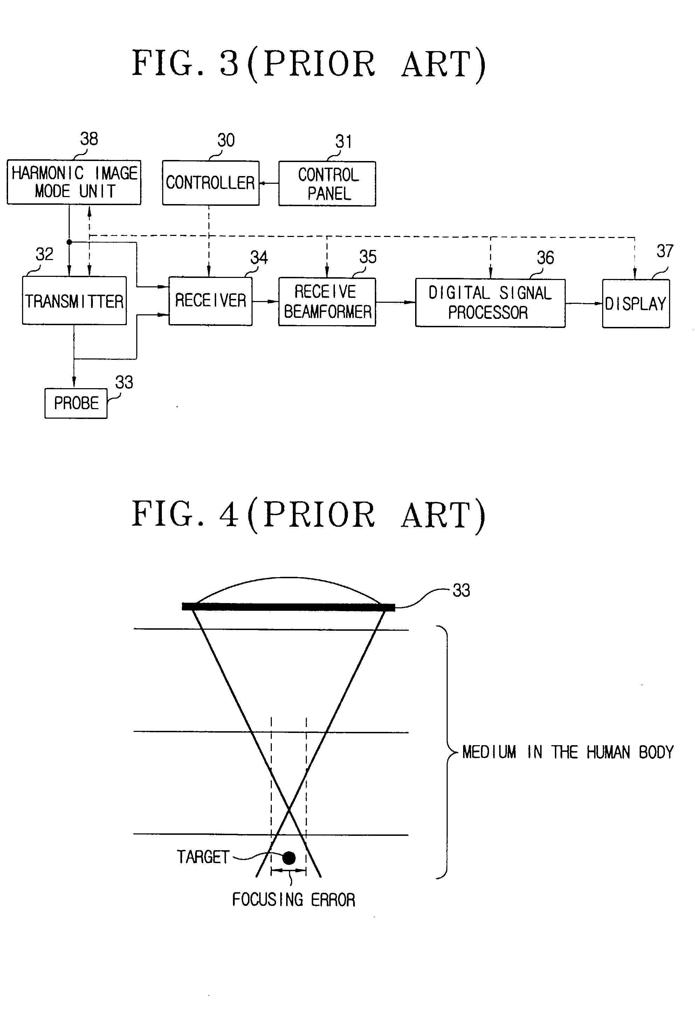

FIG. 3 is a schematic block diagram showing an ultrasonic imaging system embodying the above-described harmonic image.

In the FIG. 3 system, a controller 30 controls the entire system according to user's instructions via a control panel 31.

In a mode for realizing the fundamental image, a transmitter 32 applies a transmission pulse to the transform element of a probe 33. The probe 33 transmits an ultrasonic wave into the human body and receives a returning signal reflected from the human body. The received ultrasonic reception signal is input to a receiver 34. The receiver 34 performs a control, filtering, TGC, etc., for reception beamforming with respect to the ultrasonic reception signal. A receive beamformer 35 receives the signal-processed signal from the receiver 34 and performs a reception beamforming (dynamic focusing or synthetic focusing) of the received signal, to then output the reception beamformed signal. A digital signal processor 36 performs a signal processing in order to obtain a variety of ultrasonic images from the signal beamformed in the receive beamformer 35. A display 37 receives a signal processed signal from the digital signal processor 36 and displays an ultrasonic image with respect to the ultrasonic reception signal thereon. Here, a general ultrasonic image, that is, a fundamental image is displayed on the display 37.

During the time when a user observes a fundamental image displayed on the display 37, in the case that he or she intends to observe a harmonic image having a reduced artifact and further improved contrast and lateral resolutions, he or she presses a harmonic mode key provided on the control panel 31, in order to turn a harmonic mode on. The controller 30 controls a harmonic image mode unit 38 in correspondence to entry of the harmonic mode key. The harmonic image mode unit 38 outputs a signal for the harmonic image mode to the transmitter 32 and the receiver 34, so that a transmission pulse depending upon the velocity and frequency of a predetermined ultrasonic wave for the harmonic signal is applied to the probe 33 and the harmonic signal is processed using the ultrasonic reception signal reflected and returning from the human body. The transmitter 32 which has received the harmonic image mode signal applies the transmission pulse depending upon the velocity and frequency of the predetermined ultrasonic wave for the harmonic signal to the probe 33. The probe 33 transmits the ultrasonic wave depending upon the transmission pulse which has received from the transmitter 32 into the human body and receives the signal reflected and returning from the human body. The received ultrasonic reception signal is input to the receiver 34. The receiver 34 separates a harmonic signal from the received ultrasonic reception signal and performs the above-described operation with respect to the separated harmonic signal. The receive beamformer 35 and the digital signal processor 36 also perform the above-described operations in the same manner and the display 37 displays the harmonic image thereon.

In the above-described ultrasonic imaging system, the ultrasonic wave transmitted from the probe 33 in the human body in order to obtain the harmonic image is transmitted at a predetermined velocity as a single value without taking changes in the ultrasonic velocities depending upon the medium in the human body into consideration. As a result, as shown in FIG. 4, the ultrasonic wave transmitted from the probe 33 to the medium in the human body is not focussed on a point (target) where an ultrasonic image is desired to be obtained and does not return from the target. Instead, the ultrasonic wave is focussed at a point in front of the target and returns therefrom to the probe 33. Thus, the ultrasonic imaging system for the above-described harmonic image induces a focusing error as shown in FIG. 4.

Further, since the harmonic signal is generated in the human body. the above conventional ultrasonic imaging system is effective in reduction of an artifact at the time of receiving an ultrasonic wave, but is ineffective in reduction of an artifact at the time of transmitting an ultrasonic wave. Finally, the conventional ultrasonic imaging system cannot obtain an accurate ultrasonic image (harmonic image) with respect to the medium in the human body where an ultrasonic image is desired to be obtained.

### SUMMARY OF THE INVENTION

To solve the above problems, it is an object of the present invention to provide an ultrasonic imaging system which realizes an optimal harmonic image in which an ultrasonic velocity used for processing an ultrasonic signal, which is transmitted into a human body and is reflected therefrom is varied according to the medium in the human body from which a harmonic image is obtained.

It is another object of the present invention to provide a method for realizing an optimal harmonic image in an ultrasonic imaging system in which an ultrasonic velocity used for processing an ultrasonic signal, which is transmitted into a human body and is reflected therefrom is varied according to the medium in the human body from which a harmonic image is obtained.

To accomplish the object according to one aspect of the present invention, there is provided an ultrasonic imaging system for realizing an optimum harmonic image comprising: a transmitter/receiver unit for transmitting an ultrasonic wave to an object and receiving the returning ultrasonic signal reflected from the object; and an optimum harmonic image (OHI) realization unit for realizing an OHI by varying an ultrasonic velocity used for processing an ultrasonic signal, which is transmitted into the object and is reflected therefrom according to the medium in the object from which a harmonic image is desired to be obtained.

There is also provided a method for realizing an optimum harmonic image (OHI) in which an ultrasonic wave is transmitted to an object and a returning signal reflected from the object is received, the OHI realization method comprising the steps of: varying an ultrasonic velocity used for processing an ultrasonic signal according to the medium in the object from which a harmonic image is desired to be obtained.

### BRIEF DESCRIPTION OF THE DRAWINGS

The objects and other advantages of the present invention will become more apparent by describing in detail the tissues and operations of the present invention with reference to the accompanying drawing, in which:
Fig. 1 is a view showing a main lobe and a side lobe with respect to a fundamental signal and a harmonic signal;
FIG. 2a illustrates signal intensities of the fundamental signal from the tissue in the human body and that from the lesion in the tissue therein;
FIG. 2b illustrates signal intensities of the harmonic signal from the tissue in the human body and that from the lesion in the tissue therein;
FIG. 3 is a schematic block diagram showing a conventional ultrasonic imaging system embodying a harmonic image;
FIG. 4 shows a focusing error of a target for a harmonic image in the conventional ultrasonic imaging system;
FIG. 5 is a schematic block diagram showing an ultrasonic imaging system embodying an optimum harmonic image according to the present invention; and
FIG. 6 shows a focusing of a target for an optimum harmonic image according to the present invention.

### DETAILED DESCRIPTION OF THE PREFERRED EMBODIMENTS

A preferred embodiment of the present invention will be described with reference to the accompanying drawings.

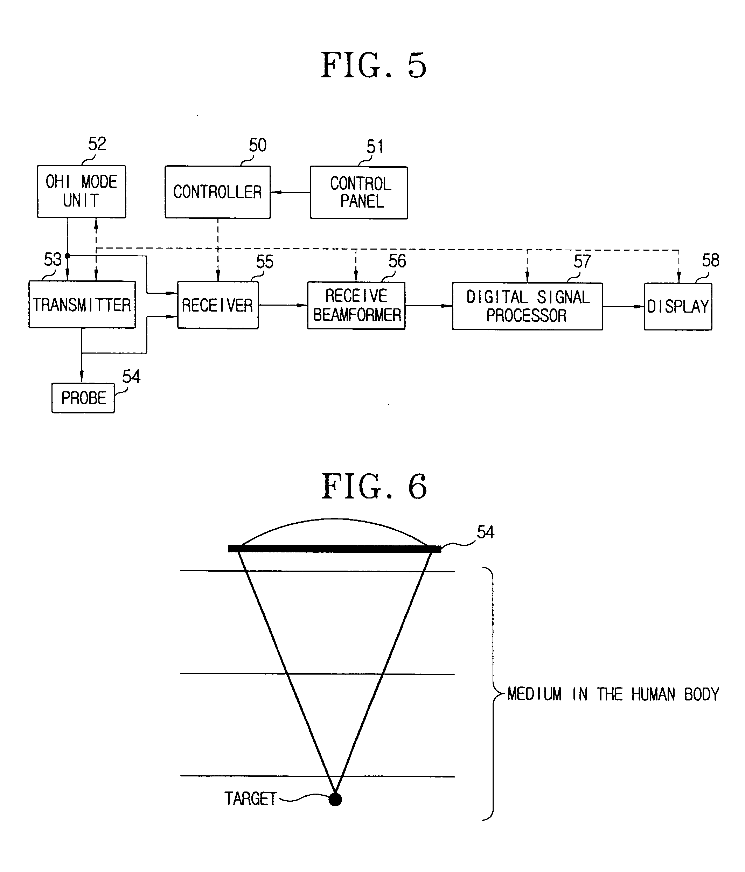

FIG. 5 is a schematic block diagram showing an ultrasonic imaging system embodying an optimum harmonic image according to the present invention. In the FIG. 5 system, the operation for a fundamental image realization mode is the same as that in the above-described FIG. 3 system. Accordingly, the detailed description thereof will be omitted herein.

Thus, during the time when a user observes a fundamental image displayed on a display 58 at the fundamental image realization mode, if the user presses a harmonic mode key provided on a control panel 51 in order to perform a harmonic mode, a controller 50 controls an optimum harmonic imaging (OHI) mode unit 52 in correspondence to entry of the harmonic mode key. The OHI mode unit 52 outputs data with respect to velocities of the ultrasonic wave depending upon the medium in the human body which are stored therein, to a digital signal processor 57. The digital signal processor 57 performs a signal processing so that the received data is displayed on a monitor and outputs the signal processed result to a display 58. The display 58 which has received the data from the digital signal processor 57 displays the velocities of the ultrasonic wave depending upon the medium in the human body.

Then, the user selects a velocity of an ultrasonic wave which is the most appropriate for the medium in the human body where a harmonic image is desired to be obtained, among the ultrasonic velocities depending upon the medium in the human body which are displayed on the display 58, using a select key provided on the control panel 51. When the velocity of the ultrasonic wave has been selected and then the select key is pressed, the OHI mode unit 52 outputs a control signal to a transmitter 53 and a receiver 55, in such a manner that a transmission pulse depending upon the selected ultrasonic velocity and a predetermined frequency is applied to a probe 54 and a harmonic signal depending upon the transmitted ultrasonic wave is separated from the ultrasonic reception signal reflected and returning from the human body, to then perform a signal processing.

The transmitter 53 which has received the control signal from the OHI mode unit 52 applies the transmission pulse depending upon the received control signal to the probe 54. The probe 54 transmits the ultrasonic wave depending upon the transmission pulse received from the transmitter 53, into the human body and receives the returning signal reflected from the human body. The received ultrasonic reception signal is input to the receiver 55. The receiver 55 separates a harmonic signal depending upon the transmitted ultrasonic wave from the ultrasonic reception signal according to the control signal applied from the OHI mode unit 52. The receiver 55 performs a control, filtering, TGC, etc., for reception beamforming with respect to the separated harmonic signal. A receive beamforrner 56 receives the signal-processed signal from the receiver 55 and performs a reception beamforming (dynamic focusing or synthetic focusing) of the received signal, to then output the reception beamformed signal. A digital signal processor 57 performs a digital signal processing in order to obtain a harmonic image from the signal beamformed in the receive beamformer 56. A display 58 receives a signal processed signal from the digital signal processor 57 and displays a harmonic image with respect to the ultrasonic reception signal thereon.

As shown in FIG. 6, the ultrasonic wave transmitted from the probe 54 into the medium in the human body, is accurately focused on a point (target) where an ultrasonic image is desired to be obtained and a reflected signal is returned to the probe 54 therefrom. Thus, an accurate ultrasonic image is obtained with respect to the target.

In this manner, an ultrasonic wave having the most appropriate velocity for the medium in the human body where a harmonic image is desired to be obtained is transmitted into the human body and then a returning signal reflected from the medium is received. As a result, an optimum harmonic image which reduces an artifact generated at the time of transmitting and receiving an ultrasonic wave and has further improved contrast and lateral resolutions can be obtained.

In addition, in the case that a user wishes to obtain a harmonic image with respect to another medium in the human body, the velocity of an ultrasonic wave which is the most appropriate for the medium in the human body is selected according to the above-described procedure, and the ultrasonic wave of the selected velocity is transmitted into the human body and reflected therefrom and then received. In this way, an optimum harmonic image is obtained with respect to the medium in the human body.

As described above, the ultrasonic imaging system for realizing the optimum harmonic image and the method therefor according to the present invention can obtain a harmonic image having a reduced artifact and further improved contrast and lateral resolutions, by varying an ultrasonic velocity used for processing an ultrasonic signal, which is transmitted into a human body and is reflected therefrom according to the medium in the human body which is desired to be obtained.

## Claims

1. An ultrasonic imaging system for realizing an optimum harmonic image comprising:
a trasmitter/receiver unit for transmitting an ultrasonic wave to an object and receiving the returning ultrasonic signal reflected from the object; and
an optimum harmonic image (OHI) realization unit for realizing an OHI by varying an ultrasonic velocity used for processing an ultrasonic signal, which is trasmitted into the object and is reflected therefrom according to the medium in the object from which a harmonic image is to be obtained.

2. The ultrasonic imaging system according to claim 1, wherein said OHI realization unit comprises:
a key input unit including a mode key for realizing a harmonic image;
an OHI mode unit which stores data with respect to the ultrasonic velocity which is varied according to the medium in the object from which the optimum harmonic image is realized according to the medium in the object, and outputs the data corresponding to the medium in the object to said trasmitter/receiver unit at a harmonic image realization mode;
a signal processor which receives the signal output from said transmitter/receiver unit and performs a signal beamforming and signal processing for realizing an optimum harmonic image; and
a controller for controlling the entire system in order to realize an ultrasonic image and the OHI in correspondence to the key entry from said key input unit.

3. The ultrasonic imaging system according to claim 2, wherein said key input unit further comprises a selection key for selecting a velocity of the ultrasonic wave which is optimal in the medium in the object from which a harmonic image is to be obtained.

4. The ultrasonic imaging system according to claim 2, wherein said controller controls said OHI mode unit so that data with respect to the selected ultrasonic velocity by the key entry via the selection key is output to said transmitter/receiver unit.

5. The ultrasonic imaging system according to claim 3, further comprising display for displaying the ultrasonic velocities which are varied according to the medium in the object in order that a user can select an ultrasonic velocity which is optimal for the medium in the object from which a harmonic image is to be obtained.

6. The ultrasonic imaging system according to claim 2, wherein said signal processor receives data with respect to the ultrasonic velocity which is varied according to the medium in the object from said OHI mode unit at a harmonic image realization mode, and performs a signal processing for displaying the received data on said display, to then output the result to said display.

7. The ultrasonic imaging system according to claims 1-6, wherein said transmitter/receiver unit receives data from said OHI mode unit, transmit the ultrasonic wave corresponding to the received data to the object, receives the signal reflected from the object, separates the harmonic signal with respect to the trasmitted ultrasonic wave from the received signal, and performs a signal processing for reception beamforming.

8. A method for realizing an optimum harmonic image (OHI) in which an ultrasonic wave is trasmitted to an object and a returning signal reflected from the object is received, the OHI realization method comprising the steps of:
varying an ultrasonic velocity used for processing an ultrasonic signal, which is transmitted into the object and is reflected therefrom according to the medium in the object from which a harmonic image is to be obtained.

9. The OHI realization method according to claim 8, further comprising the steps of:
(a) selecting the ultrasonic velocity for realizing the optimum harmonic image with respect to the medium in the object from which a harmonic image is to be obtained;
(b) trasmitting the ultrasonic wave according to the velocity selected in step (a) to the object and receiving a signal reflected from the object; and
(c)-separating the harmonic signal according to the trasmitted ultrasonic wave from the signal received in step (b) and performing a signal processing in order to realize a harmonic image.

10. The OHI realization method according to claim 9, wherein said step (a) further comprises the step of displaying ultrasonic velocities thereon so that the user can select an ultrasonic velocity.
